(19)

Europäisches Patentamt

European Patent Office

Office européen des brevets

(11) **EP 2 563 510 B1**

(12) **EUROPÄISCHE PATENTSCHRIFT**

(45) Veröffentlichungstag und Bekanntmachung des Hinweises auf die Patenterteilung:
**12.06.2019 Patentblatt 2019/24**

(21) Anmeldenummer: **11717560.4**

(22) Anmeldetag: **26.04.2011**

(51) Int Cl.:
*B01J 37/08* *(2006.01)*     *B01J 37/02* *(2006.01)*
*B01J 23/00* *(2006.01)*     *B01J 23/888* *(2006.01)*
*B01J 35/00* *(2006.01)*     *B01J 35/02* *(2006.01)*
*B01J 37/00* *(2006.01)*     *C07C 51/235* *(2006.01)*

(86) Internationale Anmeldenummer:
**PCT/EP2011/056528**

(87) Internationale Veröffentlichungsnummer:
**WO 2011/134932 (03.11.2011 Gazette 2011/44)**

(54) **SCHALENKATALYSATOR BESTEHEND AUS EINEM HOHLZYLINDRISCHEN TRÄGERKÖRPER UND EINER AUF DIE ÄUßERE OBERFLÄCHE DES TRÄGERKÖRPERS AUFGEBRACHTEN KATALYTISCH AKTIVEN OXIDMASSE**

SHELL CATALYTIC CONVERTER MADE OF A HOLLOW CYLINDRICAL CARRIER BODY AND A CATALYTICALLY ACTIVE OXIDE MASS APPLIED TO THE OUTER SURFACE OF THE CARRIER BODY

CATALYSEUR À ENVELOPPE CONSTITUÉ D'UN CORPS DE SUPPORT CYLINDRIQUE CREUX ET D'UNE MATIÈRE OXYDIQUE CATALYTIQUEMENT ACTIVE APPLIQUÉE SUR LA SURFACE EXTÉRIEURE DU CORPS DE SUPPORT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.04.2010 US 328670 P**
**10.06.2010 US 353230 P**
**10.06.2010 DE 102010023312**
**28.04.2010 DE 102010028328**

(43) Veröffentlichungstag der Anmeldung:
**06.03.2013 Patentblatt 2013/10**

(73) Patentinhaber: **BASF SE**
**67056 Ludwigshafen am Rhein (DE)**

(72) Erfinder:
• **KARPOV, Andrey**
**68159 Mannheim (DE)**
• **HORSTMANN, Catharina**
**67056 Ludwigshafen (DE)**
• **DOBNER, Cornelia Katharina**
**67071 Ludwigshafen (DE)**
• **MACHT, Josef**
**68159 Mannheim (DE)**
• **ROSOWSKI, Frank**
**68239 Mannheim (DE)**
• **MÜLLER-ENGEL, Klaus Joachim**
**76297 Stutensee (DE)**

(74) Vertreter: **BASF IP Association**
**BASF SE**
**G-FLP-C006**
**67056 Ludwigshafen (DE)**

(56) Entgegenhaltungen:
**EP-A1- 0 015 569     EP-A2- 0 714 700**
**DE-A1- 19 927 624**

Bemerkungen:
Die Akte enthält technische Angaben, die nach dem Eingang der Anmeldung eingereicht wurden und die nicht in dieser Patentschrift enthalten sind.

**Beschreibung**

Gegenstand der vorliegenden Erfindung ist ein Schalenkatalysator bestehend aus einem hohlzylindrischen Trägerkörper einer Länge von 2 bis 10 mm, einem Außendurchmesser von 4 bis 10 mm und einer Wanddicke von 1 bis 4 mm sowie einer auf die äußere Oberfläche des Trägerkörpers aufgebrachten Schale aus katalytisch aktiver Oxidmasse der allgemeinen Formel I,

$$Mo_{12}V_{2\ bis\ 4}W_{0\ bis\ 3}Cu_{0,8\ bis\ 1,5}X^1_{0\ bis\ 4}X^2_{0\ bis\ 40}O_n \qquad (I)$$

, in der die Variablen folgende Bedeutung haben:

| | |
|---|---|
| $X^1$ = | eines oder mehrere Elemente der Alkali- und Erdalkalimetalle; |
| $X^2$ = | eines oder mehrere Elemente aus der Gruppe Si, Al, Ti und Zr; und |
| $n$ = | der stöchiometrische Koeffizient des Elementes Sauerstoff, der durch die stöchiometrischen Koeffizienten der von Sauerstoff verschiedenen Elemente sowie deren Ladungszahl in I bestimmt wird. |

Ringförmige Schalenkatalysatoren aus einem ringförmigen Trägerkörper sowie einer auf die äußere Oberfläche des Trägerkörpers aufgebrachten, wenigstens die Elemente Mo, V und Cu enthaltenden, Schale aus katalytisch aktiver Oxidmasse sind bekannt (vgl. z.B. EP-A 714 700, DE-A 199 27 624 und EP0015569 oder DE-A 10360057). Sie werden hauptsächlich als Katalysatoren für die heterogen katalysierte partielle Gasphasenoxidation von Acrolein zu Acrylsäure eingesetzt. Charakteristisch ist dabei, dass in allen beispielhaften Ausführungsformen dieser ringförmigen Schalenkatalysatoren das molare Verhältnis R, gebildet aus der in der katalytisch aktiven Oxidmasse enthaltenen molaren Menge an Cu ($m_{Cu}$) und der in der katalytisch aktiven Oxidmasse enthaltenen molaren Menge an V (mv) als R = $m_{Cu}/m_V$, wenigstens 0,8 beträgt.

Nachteilig an solchen ringförmigen Schalenkatalysatoren bei deren Verwendung als Katalysatoren für die heterogen katalysierte partielle Gasphasenoxidation von Acrolein zu Acrylsäure ist jedoch, dass sie sowohl hinsichtlich der Selektivität der Acrylsäurebildung ($S^{AS}$) als auch hinsichtlich ihrer Aktivität nicht im vollen Umfang zu befriedigen vermögen.

Die Aufgabe der vorliegenden Erfindung bestand daher darin, verbesserte ringförmige Schalenkatalysatoren aus einem ringförmigen Trägerkörper sowie einer auf die äußere Oberfläche des Trägerkörpers aufgebrachten, wenigstens die Elemente Mo, V und Cu enthaltenden, Schale aus katalytisch aktiver Oxidmasse zur Verfügung zu stellen, die insbesondere bei ihrer Verwendung als Katalysatoren für die heterogen katalysierte partielle Gasphasenoxidation von Acrolein zu Acrylsäure eine verbesserte Selektivität und eine höhere Aktivität aufweisen.

Demgemäß werden ringförmige Schalenkatalysatoren, bestehend aus einem hohlzylindrischen (ringförmigen) Trägerkörper einer Länge von 2 bis 10 mm, einem Außendurchmesser von 4 bis 10 mm und einer Wanddicke von 1 bis 4 mm sowie einer auf die äußere Oberfläche des Trägerkörpers aufgebrachten Schale aus katalytisch aktiver Oxidmasse der allgemeinen Formel I,

$$Mo_{12}V_{2\ bis\ 4}W_{0\ bis\ 3}Cu_{0,8\ bis\ 1,5}X^1_{0\ bis\ 4}X^2_{0\ bis\ 40}O_n \qquad (I)$$

, in der die Variablen folgende Bedeutung haben:

| | |
|---|---|
| $X^1$ = | eines oder mehrere Elemente der Alkali- und Erdalkalimetalle; |
| $X^2$ = | eines oder mehrere Elemente aus der Gruppe Si, Al, Ti und Zr; und |
| $n$ = | der stöchiometrische Koeffizient des Elementes Sauerstoff, der durch die stöchiometrischen Koeffizienten der von Sauerstoff verschiedenen Elemente sowie deren Ladungszahl in I bestimmt wird; |

zur Verfügung gestellt.

Erfindungsgemäß vorteilhaft ist der stöchiometrische Koeffizient des Elements W in der allgemeinen Formel I 0,2 bis 3, vorzugsweise 0,5 bis 2 und besonders bevorzugt 0,75 bis 1,5.

Der stöchiometrische Koeffizient des Elements V beträgt in der allgemeinen Formel I erfindungsgemäß vorteilhaft 2,5 bis 3,5.

Der stöchiometrische Koeffizient des Elements Cu beträgt in der allgemeinen Formel I erfindungsgemäß bevorzugt 1,0 bis 1,5.

Elemente $X^1$ und $X^2$ müssen nicht in notwendiger Weise Bestandteil der katalytisch aktiven Oxidmassen der allgemeinen Formel I sein.

Elemente $X^2$ wirken innerhalb der katalytisch aktiven Oxidmassen der allgemeinen Formel I wie inerte Verdünnungsmittel. Durch ihre Einarbeitung in die katalytisch aktiven Oxidmassen der allgemeinen Formel I kann die volumenspezifische Aktivität derselben auf das gewünschte Niveau eingestellt werden. Häufig beträgt der stöchiometrische Koeffizient von $X^2$ in den erfindungsgemäß anzuwendenden katalytisch aktiven Oxidmassen der allgemeinen Formel I 0 bis 15, oder 0 bis 8. Besonders bevorzugt enthalten die erfindungsgemäß zu verwendenden katalytisch aktiven Oxidmassen der allgemeinen Formel I kein Element $X^2$. Diese Aussage trifft in entsprechender Weise auch auf die Elemente $X^1$ zu, die auf die katalytische Aktivität einen moderierenden Einfluss haben. Häufig wird der stöchiometrische Koeffizient von $X^1$ in den erfindungsgemäß anzuwendenden katalytisch aktiven Oxidmassen der allgemeinen Formel I daher 0 bis 2, oder 0 bis 1, oder 0 bis 0,2 betragen.

Erfindungsgemäß bevorzugte Schalenkatalysatoren bestehen aus einem hohlzylindrischen Trägerkörper einer Länge von 3 bis 6 mm, einem Außendurchmesser von 4 bis 8 mm und einer Wanddicke von 1 bis 2 mm sowie einer auf die äußere Oberfläche des Trägerkörpers aufgebrachten Schale aus katalytisch aktiver Oxidmasse der allgemeinen Formel I. Ganz besonders bevorzugt sind erfindungsgemäße Schalenkatalysatoren, bei denen der ringförmige (hohlzylindrische) Trägerkörper die Geometrie 7 mm x 3 mm x 4 mm (Außendurchmesser x Länge x Innendurchmesser) aufweist.

Die Dicke der bei den erfindungsgemäßen Schalenkatalysatoren auf den hohlzylindrischen Trägerkörper aufgebrachten Schale aus katalytisch aktiver Oxidmasse liegt anwendungstechnisch zweckmäßig in der Regel bei 10 bis 1000 $\mu$m. Bevorzugt beträgt diese Schalendicke bei erfindungsgemäßen Schalenkatalysatoren 10 bis 500 $\mu$m, besonders bevorzugt 100 bis 500 $\mu$m und ganz besonders bevorzugt 200 bis 300 $\mu$m.

Mit Vorteil ist die Schalendicke über einen einzelnen Schalenkatalysator betrachtet möglichst einheitlich. Bei der Herstellung einer größeren Produktionscharge erfindungsgemäßer Schalenkatalysatoren ist die Schalendicke über mehrere individuelle Schalenkatalysatorringkörper betrachtet ebenfalls möglichst einheitlich.

Der ringförmige Trägerkörper der erfindungsgemäßen Schalenkatalysatoren besteht vorzugsweise aus chemisch inertem Material. Dadurch greifen die Trägerkörper in den Ablauf der Gasphasenoxidation, die durch die erfindungsgemäßen Schalenkatalysatoren katalysiert wird, im Wesentlichen nicht ein. Als solche inerten Materialien für die Trägerkörper kommen erfindungsgemäß insbesondere Aluminiumoxid, Siliciumdioxid, Silicate wie Ton, Kaolin, Steatit, Bims, A-luminiumsilicat und Magnesiumsilicat, Siliciumcarbid, Zirkondioxid und Thoriumdioxid in Betracht (erfindungsgemäß besonders bevorzugt sind ringförmige Trägerkörper aus Steatit des Typs C 220 der Fa. CeramTec). Vorgenannte Materialien können dabei porös oder unporös sein. Bevorzugt sind Trägerkörper mit deutlich ausgebildeter Oberflächenrauhigkeit (z.B. Hohlzylinder mit Splittauflage).

Die Herstellung erfindungsgemäßer Schalenkatalysatoren kann auf unterschiedliche Art und Weise erfolgen.

Beispielsweise kann zunächst die katalytisch aktive Oxidmasse der allgemeinen Formel I als solche hergestellt werden. Eine solche Herstellung erfolgt üblicherweise dadurch, dass man von geeigneten Quellen der elementaren Konstituenten der katalytisch aktiven Oxidmasse ein möglichst inniges, vorzugsweise feinteiliges, ihrer Stöchiometrie entsprechend zusammengesetztes, Trockengemisch (eine Vorläufermasse) erzeugt und dieses bei Temperaturen von 350 bis 600°C calciniert (thermisch behandelt). Die Calcination kann sowohl unter Inertgas als auch unter einer oxidativen Atmosphäre wie z.B. Luft (oder ein anderes Gemisch aus Inertgas und Sauerstoff) sowie auch unter reduzierender Atmosphäre (z.B. Gemische aus Inertgas und reduzierenden Gasen wie $H_2$, $NH_3$, CO, Methan und/oder Acrolein oder die genannten reduzierend wirkenden Gase für sich) durchgeführt werden. Die resultierende katalytische Aktivität zeigt in Abhängigkeit vom Sauerstoffgehalt der Calcinationsatmosphäre in der Regel ein Optimum. Vorzugsweise beträgt der Sauerstoffgehalt der Calcinationsatmosphäre 0,5 bis 10 Vol.-%, besonders bevorzugt 1 bis 5 Vol.-%. Sauerstoffgehalte oberhalb und unterhalb der vorgenannten Grenzen verringern die resultierende katalytische Aktivität normalerweise. Die Calcinationsdauer kann einige Minuten bis einige Stunden betragen und nimmt üblicherweise mit der Höhe der Calcinationstemperatur ab. Ein erfindungsgemäß gut geeignetes Calcinationsverfahren beschreibt z.B. die WO 95/11081.

Als Quellen für die elementaren Konstituenten der katalytisch aktiven Oxidmasse der allgemeinen Formel I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind. Das innige Vermischen der Ausgangsverbindungen (Quellen) kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird.

Als Lösungsmittel wird bevorzugt Wasser eingesetzt. Anschließend wird die erhaltene flüssige (z. B. wässrige) Masse getrocknet, wobei der Trocknungsprozeß vorzugsweise durch Sprühtrocknung der flüssigen (z. B. wässrigen) Mischung mit Austrittstemperaturen von 100 bis 150°C erfolgt. Der trocknende Gasstrom ist anwendungstechnisch zweckmäßig Luft oder molekularer Stickstoff.

Die nach dem Calcinieren erhaltene katalytisch aktive Oxidmasse wird anschließend z.B. durch Mahlen in ein feinteiliges Pulver überführt, das dann normalerweise mit Hilfe eines flüssigen Bindemittels auf die äußere Oberfläche des Trägerkörpers aufgebracht werden kann. Die Feinheit der auf die Oberfläche des Trägerkörpers aufzubringenden katalytisch aktiven Oxidmasse wird dabei selbstredend an die gewünschte Schalendicke angepasst.

Beispielsweise werden die Trägerkörper mit dem flüssigen Bindemittel durch z.B. Besprühen kontrolliert befeuchtet und die so befeuchteten Trägerkörper mit der feinteiligen katalytisch aktiven Oxidmasse bestäubt (vgl. z.B. EP-A 714700). Anschließend wird die Haftflüssigkeit aus dem mit aktiver Oxidmasse beschichteten befeuchteten Trägerkörper wenigstens teilweise entfernt (z.B. durchleiten von heißem Gas; vgl. WO 2006/094766). Es können aber auch alle anderen in der EP-A 714700 als Stand der Technik gewürdigten Aufbringungsverfahren zur Herstellung erfindungsgemäßer Schalenkatalysatoren angewendet werden. Als flüssige Bindemittel kommen z.B. Wasser und wässrige Lösungen in Betracht.

Grundsätzlich kann zur Herstellung erfindungsgemäßer Schalenkatalysatoren aber auch so vorgegangen werden, dass man auf die Oberfläche des Trägerkörpers zunächst feinteilige Vorläufermasse aufbringt, und die Calcination der Vorläufermasse zur katalytisch aktiven Oxidmasse der allgemeinen Formel I erst nachträglich, d.h., bereits auf der Oberfläche des Trägerkörpers befindlich, durchführt.

Erfindungsgemäß bevorzugt wird man erfindungsgemäße Katalysatoren gemäß der in der EP-A 714700 beschriebenen und beispielhaft ausgeführten Herstellweise erzeugen. Eine wässrige Lösung aus 75 Gew.-% Wasser und 25 Gew.-% Glycerin ist bevorzugtes Bindemittel. Das Verfahren der thermischen Behandlung der Vorläufermasse einer katalytisch aktiven Oxidmasse wird man erfindungsgemäß vorteilhaft gemäß der in der DE-A 10360057 beschriebenen und beispielhaft ausgeführten Verfahrensweise durchführen. Dabei werden die gleichen Quellen der elementaren Konstituenten wie in der DE-A 10360057 eingesetzt, jedoch in der erfindungsgemäßen Stöchiometrie entsprechenden Mengenverhältnissen. Das erfindungsgemäß bevorzugte Aufbringungsverfahren der katalytisch aktiven Oxidmasse der allgemeinen Formel I auf die Oberfläche der hohlzylindrischen Trägerkörper ist in den Ausführungsbeispielen 1 und 2 der DE-A 10360057 ebenfalls beschrieben.

Insgesamt erfolgt die Herstellung erfindungsgemäßer Schalenkatalysatoren ganz besonders bevorzugt so wie in den Ausführungsbeispielen 1 und 2 der DE-A 10360057 beschrieben. Als Quellen für den elementaren Konstituenten Cu kommen für die Herstellung erfindungsgemäßer Katalysatoren insbesondere Kupfer(II)sulfatpentahydrat, Kupfer(II)nitrathydrat (Cu-Gehalt = 26,1 Gew.-%) und Kupfer(II)acetatmonohydrat in Betracht, unter denen die letztere bevorzugt ist. Ammoniummetavanadat ist bevorzugte Vanadinquelle und Ammoniumparawolframatheptahydrat ist bevorzugte Wolframquelle. Als Mo-Quelle wird anwendungstechnisch zweckmäßig Ammoniumheptamolybdattetrahydrat verwendet. Im Übrigen kommen als Quellen der elementaren Konstituenten neben Oxiden ganz generell vor allem Halogenide, Nitrate, Formiate, Oxalate, Acetate, Carbonate und Hydroxide in Betracht.

Erfindungsgemäße Schalenkatalysatoren (insbesondere alle in dieser Schrift beispielhaft hergestellten Schalenkatalysatoren) eignen sich insbesondere als Katalysatoren für die heterogen katalysierte partielle Gasphasenoxidation von Acrolein zu Acrylsäure und zeichnen sich dabei insbesondere durch eine verbesserte Selektivität $S^{AS}$ der Acrylsäurebildung und durch eine erhöhte Aktivität aus.

Vor diesem Hintergrund eignen sich erfindungsgemäße Schalenkatalysatoren nicht zuletzt besonders gut im Fall von Acrolein-zu-Acrylsäure-Partialoxidationen, die bei hohen Acroleinbelastungen der Katalysatorbeschickung (z.B. ≥135 Nl/l·h bis 350 bzw. bis 250 Nl/l·h) durchgeführt werden. Selbstredend können sie aber auch schon bei entsprechenden Acroleinbelastungen von ≥ 50 Nl/l·h eingesetzt werden.

Der Begriff der Belastung wird dabei so wie in der DE-A 19927624 definiert verwendet.

Vorteilhaft werden Acrolein-zu-Acrylsäure-Partialoxidationen mit erhöhter Acroleinbelastung der Katalysatorbeschickung unter Verwendung erfindungsgemäßer Schalenkatalysatoren auch so wie in der DE-A 19927624 bzw. wie in der DE-A 10360057 beschrieben durchgeführt. Erfindungsgemäß vorteilhaft wird die Katalysatorbeschickung dabei so gestaltet, dass die volumenspezifische Aktivität des Katalysatorfestbetts in Strömungsrichtung des Reaktionsgasgemischs zunimmt (vorzugsweise durch abnehmende Verdünnung der erfindungsgemäßen Schalenkatalysatoren mit inerten Verdünnungsformkörpern).

Als Reaktor zur Durchführung der heterogen katalysierten partiellen Gasphasenpartialoxidation von Acrolein zu Acrylsäure wird anwendungstechnisch zweckmäßig ein Rohrbündelreaktor eingesetzt, dessen Reaktionsrohre mit dem Katalysatorfestbett befüllt sind (vgl. DE-A 19927624). Beim Einbringen von erfindungsgemäßen Schalenkatalysatoren in die Rohre des Rohrbündelreaktors wird man zweckmäßigerweise der Lehre der DE-A 102007028333 sowie der Lehre der WO 2006/094766 folgen.

Unter der Selektivität der Acrylsäurebildung ($S^{AS}$ (mol-%)) wird in dieser Schrift verstanden:

$$S^{AS} = \frac{\text{Molzahl Acrolein umgesetzt zu Acrylsäure}}{\text{Molzahl Acrolein insgesamt umgesetzt}} \times 100.$$

(die Umsatzzahlen jeweils bezogen auf einen einmaligen Durchgang des Reaktionsgasgemischs durch das Katalysatorfestbett).

Eine unter ansonsten unveränderten Reaktionsbedingungen bei tieferer Temperatur zum gleichen Umsatz führende Aktivmasse (führender Katalysator) verfügt über eine höhere Aktivität.

Der Umsatz $U^A$ an Acrolein (mol-%) ist in entsprechender Weise definiert als:

$$U^A = \frac{\text{Molzahl umgesetztes Acrolein}}{\text{Molzahl eingesetztes Acrolein}} \times 100.$$

Beispiele und Vergleichsbeispiele

A) Herstellung von Schalenkatalysatoren

Vergleichsbeispiel 1A

Ringförmiger Schalenkatalysator V1A mit der katalytisch aktiven Oxidmasse $Mo_{12}V_3W_{1,2}Cu_{2,4}O_n$

259 g Kupfer(II)sulfatpentahydrat (Cu-Gehalt = 25,6 Gew.-%) wurden innerhalb 1 h bei 70°C in 2000 g Wasser zu einer Lösung I gelöst.

In 7000 g Wasser wurden innerhalb von 15 Minuten bei 95°C nacheinander 135 g Ammoniumparawolframatheptahydrat (W-Gehalt = 71 Gew.-%), 153 g Ammoniummetavanadat (V-Gehalt = 43,5 Gew.-%) und 920 g Ammoniumheptamolybdattetrahydrat (Mo-Gehalt = 54,5 Gew.-%) zu einer Lösung II gelöst. Anschließend wurde diese Lösung II innerhalb von 3 min. auf 98°C erhitzt. Danach wurde die 70°C warme Lösung I innerhalb von 5 Minuten sukzessive in die 98°C warme Lösung II eingerührt. Die dabei resultierende wässrige Suspension hatte eine Temperatur von 95°C und wurde 5 Minuten bei dieser Temperatur nachgerührt. Die Suspension wurde anschließend bei einer Eingangstemperatur von 330°C und einer Austrittstemperatur von 106°C im Luftstrom innerhalb von 2 h sprühgetrocknet (Sprühturm von Fa. NIRO, Sprühkopf-Nr. F0 A1). Während der Sprühtrocknung wurde der jeweils noch nicht gesprühte Anteil der Suspension unter Beibehaltung der 95°C weitergerührt. 900 g des resultierenden und auf 25°C abgekühlten Sprühpulvers wurden mit 180 g einer 50 gew.-%igen wässrigen Essigsäurelösung und zusätzlichen 170 g Wasser, die beide eine Temperatur von 25°C aufwiesen, mit einem Kneter der Firma Werner & Pfleiderer vom Typ ZS1-80 verknetet (Knetdauer: ca. 2 Stunden; Knettemperatur: 30 - 35°C).

Anschließend wurde das Knetgut mit einer Schichtdicke von 2 cm bei einer Temperatur von 110°C während 16 h im Umlufttrockenschrank getrocknet.

700 g der aus dem Trockenschrank entnommenen Vorläufermasse wurden in einem Drehrohrofen diskontinuierlich calciniert. Der Drehrohrofen bestand aus einem Ofen der Länge 162 cm und einem durch diesen Ofen durchgeführten Drehrohr der Länge 209 cm. Der Innendurchmesser des Drehrohres betrug 12,5 cm. Die oberen und unteren 23,5 cm des Drehrohrs ragten aus dem Ofen heraus. Während der gesamten Calcination (einschließlich Abkühlung) wurde durch das Drehrohr ein Gasstrom von 240 Nl/h (die Nl beziehen sich auf 25°C und 1 bar) geleitet, der aus einem Gemisch von Luft mit molekularem Stickstoff bestand und dessen Gehalt an molekularem Sauerstoff 1,9 Vol.-% betrug. Der Gasstrom wurde dem Drehrohrofen mit einer Temperatur von 25°C zugeführt. Das Drehrohr selbst war aus Edelstahl gefertigt und der Neigungswinkel des Drehrohres zur Horizontalen betrug = 1,7°. Das Drehrohr rotierte mit 1 Umdrehung/min. Haltegitter hielten das Calcinationsgut mittig auf einer Länge von ¼ der Gesamtlänge des Drehrohrs.

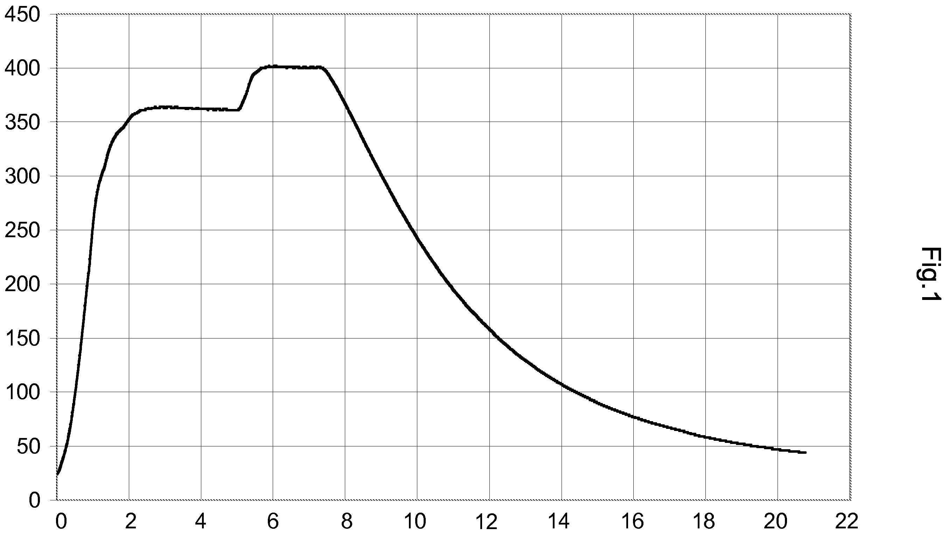

Im Rahmen der Calcinierung wurde die Vorläufermasse zunächst innerhalb von einer Stunde und 15 Minuten von 25°C im Wesentlichen linear auf eine Materialguttemperatur von 300±2°C, dann innerhalb von 45 Minuten im Wesentlichen linear auf eine Materialguttemperatur von 350±2°C und anschließend innerhalb von 30 Minuten im Wesentlichen linear auf eine Materialguttemperatur von 362±2°C erhitzt. Während der nachfolgenden 2 Stunden und 35 Minuten wurde diese Materialguttemperatur beibehalten. Dann wurde die Vorläufermasse innerhalb von 25 min. zunächst im Wesentlichen linear auf eine Materialguttemperatur von 395±2°C und daran anschließend innerhalb von weiteren 10 min. im Wesentlichen linear auf eine Materialguttemperatur von 400±2°C erwärmt, bei dieser Temperatur während einer weiteren Stunde und 45 Minuten gehalten und dann durch Abschalten des Ofens sowie unter Beibehaltung der Rotation des Drehrohrs innerhalb von ca. 13 h auf 44±2°C abgekühlt und mit dieser Temperatur aus dem Drehrohr entnommen. Figur 1 zeigt den gemessenen Verlauf der Materialguttemperatur (in °C als Ordinate) in Abhängigkeit von der Calcinationsdauer (in Stunden (h) als Abszisse).

Die dem Drehrohrofen entnommene katalytisch aktive Oxidmasse wurde anschließend in einer Mühle ZM 200 der Fa. Retsch zu einem feinteiligen Pulver gemahlen, von dem 50% der Pulverpartikel ein Sieb der Maschenweite 1 bis 10 $\mu$m passierten und dessen numerischer Anteil an Partikeln mit einer Längstausdehnung oberhalb von 50 $\mu$m

weniger als 1% betrug.

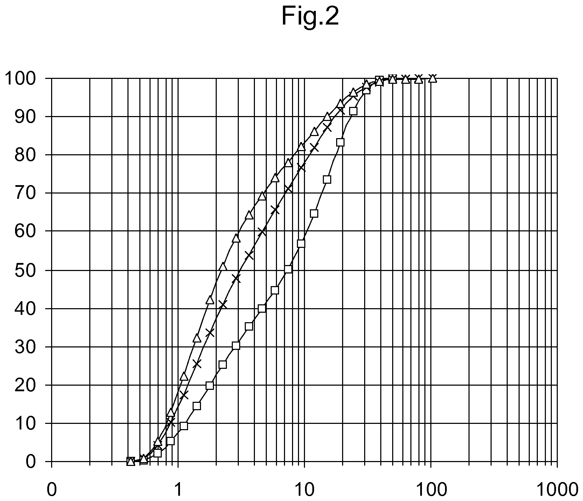

Eine für die Vergleichsbeispiele und Ausführungsbeispiele dieser Schrift günstige Größenverteilung der Partikel des vorgenannten gemahlenen katalytisch aktiven Oxidmassenpulvers zeigt die Figur 2 dieser Anmeldung (die Abszisse zeigt in logarithmischer Auftragung (im logarithmischen Maßstab) den Partikeldurchmesser (die Partikelausdehnung) in $\mu$m und die Ordinate zeigt den jeweils zugehörigen kumulativen Anteil der Partikel in Vol.-% (der Ordinatenwert eines Punktes auf der Verteilungskurve zeigt die X-% des Gesamtpartikelvolumens, die aus Partikeln mit der diesem Punkt auf der Abszisse zugeordneten Partikelausdehnung oder mit einer kleineren Partikelausdehnung bestehen; das heißt, (100 - X)% des Gesamtpartikelvolumens bestehen aus Partikeln mit einer größeren Partikelausdehnung (mit einem größeren Partikeldurchmesser)). Die zugrunde liegende Meßmethode ist dabei die Laserbeugung. Dabei wird das jeweilige feinteilige Pulver über eine Dispergierrinne in den Trockendispergierer Sympatec RO-DOS (Sympatec GmbH, System-Partikel-Technik, Am Pulverhaus 1, D-38678 Clausthal-Zellerfeld) geführt, dort mit Druckluft trocken dispergiert und im Freistrahl in die Messzelle geblasen. In dieser wird dann nach ISO 13320 mit dem Laserbeugungsspektrometer Malvern Mastersizer S (Malvern Instruments, Worcetshire WR 14 1AT, United Kingdom) die volumenbezogene Partikeldurchmesserverteilung bestimmt (obscuration 3-7%). Die Stärke der Dispergierung des Trockenpulvers während der Messung bestimmt der angewandte Dispergierdruck (Figur 2: Vierecke = 1,2 bar; Kreuze = 2,0 bar; Dreiecke = 3,5 bar; jeweils als Absolutdruck) der als Treibgas angewandten Druckluft.

Mit dem gemahlenen katalytisch aktiven Oxidmassenpulver wurden wie in S1 der EP-B 714700 beschrieben 800 g ringförmige Trägerkörper (7 mm Außendurchmesser, 3 mm Länge, 4 mm Innendurchmesser, Steatit C 220 der Fa. CeramTec mit einer Oberflächenrauhigkeit Rz von 45 $\mu$m (Splittauflage)) beschichtet. Das auf das Volumen der Trägerkörpermasse bezogene Porengesamtvolumen des Trägerkörpers betrug $\leq$ 1 Vol.-%. Bindemittel war eine wässrige Lösung aus 75 Gew.-% Wasser und 25 Gew.-% Glycerin. Die Beschichtung erfolgte in einer rotierenden Dragiertrommel (Innendurchmesser = 25,5 cm; 36 U/min), die mit den Trägerkörpern befüllt worden war. Ca. 60 mL des flüssigen Bindemittels wurden über eine Düse (Düsendurchmesser = 1 mm) innerhalb von 60 min. auf die Trägerkörper aufgesprüht (die exakte Bindemittelmenge wurde jeweils so bemessen, dass keine Ausbildung von Zwillingen erfolgte, aber die gesamte Pulvermenge auf die Oberfläche der Trägerkörper aufgenommen wurde ohne dass Pulveragglomeration eintrat). Gleichzeitig wurden im selben Zeitraum 205 g des gemahlenen katalytisch aktiven Oxidmassenpulvers mittels einer Förderschnecke außerhalb des Sprühkegels der Zerstäuberdüse kontinuierlich zudosiert. Während der Beschichtung wurde das zugeführte Pulver vollständig auf die Oberfläche der Trägerkörper aufgenommen. Eine Agglomeration der feinteiligen oxidischen Aktivmasse wurde nicht beobachtet.

Anschließend wurden die beschichteten Ringe 2 h bei einer Temperatur von 300°C in einem Umlufttrockenschrank gehalten (entfeuchtet).

Die dem Umlufttrockenschrank entnommenen Schalenkatalysatoren wiesen, bezogen auf ihre Gesamtmasse, einen oxidischen Aktivmassenanteil von ca. 20 Gew.-% auf. Die Aktivmassenschalendicke betrug 150 bis 250 $\mu$m.

Vergleichsbeispiel V2A

Die Herstellung des ringförmigen Schalenkatalysators V2A mit der katalytisch aktiven Oxidmasse $Mo_{12}V_3W_{1,2}Cu_{2,4}O_n$, erfolgte wie bei V1A, anstelle der 259 g Kupfer(II)sulfatpentahydrat wurden jedoch 255 g Kupfer(II)nitrathydrat (Cu-Gehalt = 26,1 Gew.-%) als Cu-Quelle verwendet.

Vergleichsbeispiel V3A

Die Herstellung des ringförmigen Schalenkatalysators V3A mit der katalytisch aktiven Oxidmasse $Mo_{12}V_3W_{1,2}Cu_{2,4}O_n$, erfolgte wie bei V1A, anstelle der 259 g Kupfer(II)sulfatpentahydrat wurden jedoch 210 g Kupfer(II)acetatmonohydrat (Cu-Gehalt = 31,7 Gew.-%) als Cu-Quelle verwendet.

Ausführungsbeispiel 1A

Die Herstellung des ringförmigen Schalenkatalysators A1A mit der katalytisch aktiven Oxidmasse $Mo_{12}V_3W_{1,2}Cu_{1,2}O_n$ erfolgte wie bei V1A, es wurden jedoch 134,0 g Kupfer(II)sulfatpenta-hydrat, 139,7 g Ammoniumparawolframatheptahydrat, 158 g Ammoniummetavanadat und 950 g Ammoniumheptamolybdattetrahydrat eingesetzt.

Ausführungsbeispiel 2A

Die Herstellung des ringförmigen Schalenkatalysators A2A mit der katalytisch aktiven Oxidmasse $Mo_{12}V_3W_{1,2}Cu_{1,2}O_n$ erfolgte wie bei V2A, es wurden jedoch 127,3 g Kupfer(II)nitrattrihydrat als Cu-Quelle verwendet.

Ausführungsbeispiel 3A

Die Herstellung des ringförmigen Schalenkatalysators A3A mit der katalytisch aktiven Oxidmasse $Mo_{12}V_3W_{1,2}Cu_{1,2}O_n$ erfolgte wie bei V3A, es wurden jedoch 104,8 g Kupfer(II)acetatmono-hydrat als Cu-Quelle verwendet.

B) Testung der Schalenkatalysatoren V1A bis A3A als Katalysatoren für die heterogen katalysierte partielle Gasphasenoxidation von Acrolein zu Acrylsäure

Ein Reaktionsrohr (V2A Stahl; 30 mm Außendurchmesser; 2 mm Wandstärke; 26 mm Innendurchmesser; 440 cm Länge; ein in der Mitte des Reaktionsrohrquerschnitts zentriertes und im Reaktionsrohr längs zu selbigem geführtes Thermorohr (4 mm Außendurchmesser) zur Aufnahme eines Thermoelements) wurde von unten nach oben jeweils wie folgt beschickt:

Abschnitt 1: 25 cm Länge
Katalysatorstuhl aus V2A Stahl zur Aufnahme des Katalysatorfestbetts;

Abschnitt 2: 55 cm Länge
Vorschüttung aus Steatit-Kugeln mit einem Durchmesser von 4 bis 5 mm (Steatit C220 der Fa. CeramTec);

Abschnitt 3: 100 cm Länge
Katalysatorfestbettschüttung aus einem homogenen Gemisch bestehend aus 30 Gew.-% Steatit-Ringen der Geometrie 7 mm x 3 mm x 4 mm (Außendurchmesser x Länge x Innendurchmesser; Steatit C220 der Fa. CeramTec) und 70 Gew.-% des jeweiligen Schalenkatalysators;

Abschnitt 4: 200 cm Länge
Katalysatorfestbettschüttung ausschließlich bestehend aus dem in Abschnitt 3 jeweils verwendeten Schalenkatalysator;

Abschnitt 5: 60 cm Leerrohr.

Durch das jeweilige wie vorstehend beschrieben beschickte Reaktionsrohr wurde von unten nach oben durch das Reaktionsrohr strömend ein Reaktionsgasgemisch geführt, das folgende Gehalte aufwies:

4,6 Vol.-% Acrolein,
0,1 Vol.-% Propen,
0,2 Vol.-% Acrylsäure,
5,4 Vol.-% $O_2$,
1,6 Vol.-% CO und $CO_2$,
81,9 Vol.-% $N_2$ und
6,2 Vol.-% $H_2O$.

Die Zufuhrtemperatur des Reaktionsgasgemischs (am Eingang ins Reaktionsrohr) betrug 210 °C und die Belastung des Katalysatorfestbetts (wie in der DE-A 19927624 definiert) mit Acrolein war 85 Nl/l·h.

Die Temperierung des Reaktionsrohres erfolgte über die Länge desselben (bis auf die letzten 20 cm des Rohres im Abschnitt 1 und die 60 cm Leerrohr im Abschnitt 5) jeweils mittels eines nicht umgepumpten sondern mit molekularem Stickstoff nach dem Prinzip der Mammutpumpe durchperlten und von außen elektrisch beheizten, die jeweils erforderliche Salzbadtemperatur $T^B$ (°C) über die Zeit und die Rohrlänge konstant aufweisenden Salzbades (53 Gew.-% Kaliumnitrat, 40 Gew.-% Natriumnitrit und 7 Gew.-% Natriumnitrat, 220 kg Salzschmelze). Der in das Salzbad mit einer Temperatur von 25°C von unten eingeperlte Stickstoffstrom betrug 300 Nl/h (die Einperlung erfolgte über sechs über den Querschnitt des Salzbades gleichmäßig verteilte Düsen mit 50 Nl/h je Düse). Die Salzbadtemperatur $T^B$ (°C) wurde in allen Fällen so eingestellt, dass ein auf den einfachen Durchgang des Reaktionsgasgemischs durch das Katalysatorfestbett bezogener Acroleinumsatz von ca. 99,5 mol-% resultierte. Entlang des Reaktionsrohres änderte sich die Salzbadtemperatur infolge Zuheizung nicht (es wurde vom Salzbad mehr Wärme abgegeben wie vom Reaktionsrohr an das Salzbad übertragen).

Die nachfolgende Tabelle 1 zeigt die in Abhängigkeit vom eingesetzten Schalenkatalysator nach jeweils 100 Betriebsstunden resultierenden Ergebnisse:

Tabelle 1

| Schalenkatalysator | $T^B$ (°C) | $U^A$ (mol-%) | $S^{AS}$ (mol-%) |
|---|---|---|---|
| V1A | 261 | 99,4 | 95,1 |
| V2A | 294 | 99,5 | 95,6 |
| V3A | 258 | 99,4 | 96,0 |
| A1A | 251 | 99,6 | 96,8 |
| A2A | 264 | 99,5 | 97,3 |
| A3A | 257 | 99,5 | 97,5 |

Die in Tabelle 1 gezeigten Ergebnisse weisen aus, dass unabhängig von der eingesetzten Cu-Quelle die Stöchiometrie $Mo_{12}V_3W_{1,2}Cu_{1,2}O_n$ der katalytisch aktiven Oxidmasse im Vergleich zur Stöchiometrie $Mo_{12}V_3W_{1,2}Cu_{2,4}O_n$ markant höhere Selektivitäten der Acrylsäurebildung bedingt.

Darüber hinaus zeigen die Ergebnisse in Tabelle 1, dass die besten $S^{AS}$-Werte bei Verwendung von Kupfer(II)acetatmonohydrat als Cu-Quelle erhalten werden.

Im Übrigen zeigen die erfindungsgemäßen Schalenkatalysatoren vergleichsweise erhöhte Aktivitäten.

C) Herstellung von Schalenkatalysatoren

Vergleichsbeispiel 1B

Ringförmiger Schalenkatalysator V1B mit der katalytisch aktiven Oxidmasse $Mo_{12}V_3W_{1,2}Cu_{0,6}O_n$

23,9 g Kupfer(II)acetatmonohydrat (Cu-Gehalt = 31,7 Gew.-%) wurden unter Rühren bei 25°C in 1600 g Wasser zu einer Lösung I gelöst.

In 3780 g 90°C warmem Wasser wurden innerhalb von 5 min. 420 g Ammoniumheptamolybdattetrahydrat (Mo-Gehalt = 54,5 Gew.-%) gelöst. Anschließend wurden unter Beibehalt der 90°C 69,1 g Ammoniummetavanadat (V-Gehalt = 43,5 Gew.-%) zugegeben und die resultierende Lösung bei 90°C weitere 30 Minuten nachgerührt. Danach wurden 61,8 g Ammoniumparawolframatheptahydrat (W-Gehalt = 71 Gew.-%) zugegeben und die resultierende Lösung II bei 90°C 40 Minuten nachgerührt. Anschließend wurde die Lösung II innerhalb von 10 Minuten auf 80°C abgekühlt.

Die 25°C warme Lösung I wurde innerhalb von 5 Minuten in die 80°C warme Lösung II eingerührt. Das resultierende Gemisch hatte eine Temperatur von 75°C. Dann wurden 765 g einer 25 gew.-%igen wässrigen $NH_3$-Lösung, deren Temperatur 25°C betrug, innerhalb von 15 min. zu dem 75°C warmen Gemisch zugesetzt. Die dabei resultierende wässrige Lösung hatte eine Temperatur von 79°C und wurde innerhalb von 2 min. auf 80°C erhitzt und 10 Minuten bei dieser Temperatur nachgerührt. Die Lösung wurde anschließend bei einer Eingangstemperatur von 350°C und einer Austrittstemperatur von 120°C innerhalb von 2 h im Luftstrom sprühgetrocknet (Sprühturm von Fa. NIRO, Sprühkopf-Nr. F0 A1). Während der Sprühtrocknung wurde der jeweils noch nicht gesprühte Anteil der Suspension unter Beibehaltung der 80°C weitergerührt.

900 g des resultierenden und auf 25°C abgekühlten Sprühpulvers wurden mit 180 g einer 50 gew.-%igen wässrigen Essigsäurelösung und zusätzlichen 90 g Wasser, die beide eine Temperatur von 25°C aufwiesen, mit einem Kneter der Fa. Werner & Pfleiderer vom Typ ZS1-80 verknetet (Knetdauer: ca. 2 Stunden; Knettemperatur: 30 - 35°C).

Anschließend wurde das Knetgut mit einer Schichtdicke von ca. 2 cm bei einer Temperatur von 110°C während 16 h im Umlufttrockenschrank getrocknet.

700 g der aus dem Trockenschrank entnommenen Vorläufermasse wurden in einem Drehrohrofen calciniert. Der Drehrohrofen bestand aus einem Ofen der Länge 162 cm und einem durch diesen Ofen durchgeführten Drehrohr der Länge 209 cm. Der Innendurchmesser des Drehrohres betrug 12,5 cm. Die oberen und unteren 23,5 cm des Drehrohrs ragten aus dem Ofen heraus. Während der gesamten Calcination (einschließlich Abkühlung) wurde durch das Drehrohr ein Gasstrom von 240 Nl/l (die Nl beziehen sich auf 25°C und 1 bar) geleitet, der aus einem Gemisch von Luft mit molekularem Stickstoff bestand und dessen Gehalt an molekularem Sauerstoff 2,2 Vol.-% betrug. Der Gasstrom wurde dem Drehrohrofen mit einer Temperatur von 25°C zugeführt. Das Drehrohr selbst war aus Edelstahl gefertigt und der Neigungswinkel des Drehrohres zur Horizontalen betrug 1,7°. Das Drehrohr rotierte mit 1 Umdrehung/min. Haltegitter hielten das Calcinationsgut mittig auf einer Länge von ¼ der Gesamtlänge des Drehrohres.

Im Rahmen der Calcinierung wurde die Vorläufermasse zunächst innerhalb von einer Stunde und 15 Minuten von 25°C im Wesentlichen linear auf eine Materialguttemperatur von 300±2°C, dann innerhalb von 45 Minuten im Wesentlichen linear auf eine Materialguttemperatur von 350±2°C und anschließend innerhalb von 30 Minuten im Wesentlichen linear auf eine Materialguttemperatur von 362±2°C erhitzt. Während der nachfolgenden 2 Stunden und 35 Minuten wurde diese Materialguttemperatur beibehalten. Dann wurde die Vorläufermasse innerhalb von 25 min. zunächst im Wesentlichen linear auf eine Materialguttemperatur von $395 \pm 2°C$ und daran anschließend innerhalb von weiteren 10 min. im Wesentlichen linear auf eine Materialguttemperatur von $400 \pm 2°C$ erwärmt, bei dieser Temperatur während einer weiteren Stunde und 45 Minuten gehalten und dann durch Abschalten des Ofens sowie unter Beibehaltung der Rotation des Drehrohrs innerhalb von ca. 13 h auf $44 \pm 2°C$ abgekühlt und mit dieser Temperatur aus dem Drehrohr entnommen. Figur 1 zeigt den Verlauf der Materialguttemperatur in Abhängigkeit von der Calcinationsdauer.

Die dem Drehrohrofen entnommene katalytisch aktive Oxidmasse wurde anschließend in einer Mühle ZM 200 der Fa. Retsch zu einem feinteiligen Pulver gemahlen, von dem 50% der Pulverpartikel ein Sieb der Maschenweite 1 bis 10 $\mu$m passierten und dessen numerischer Anteil an Partikeln mit einer Längsausdehnung oberhalb von 50 $\mu$m weniger als 1% betrug.

Mit dem gemahlenen katalytisch aktiven Oxidmassenpulver wurden wie in S1 der EP-B 714700 beschrieben 800 g ringförmige Trägerkörper (7 mm Außendurchmesser, 3 mm Länge, 4 mm Innendurchmesser, Steatit C 220 der Fa. CeramTec mit einer Oberflächenrauhigkeit Rz von 45 $\mu$m (Splittauflage)) beschichtet. Das auf das Volumen der Trägerkörpermasse bezogene Porengesamtvolumen des Trägerkörpers betrug ≤ 1 Vol.-%. Bindemittel war eine wässrige Lösung aus 75 Gew.-% Wasser und 25 Gew.-% Glycerin. Die Beschichtung erfolgte in einer rotierenden Dragiertrommel (36 U/min), die mit den Trägerkörpern befüllt worden war. Ca. 90 mL des flüssigen Bindemittels wurden über eine Düse (Düsendurchmesser = 1 mm) innerhalb von 60 min. auf die Trägerkörper aufgesprüht (die exakte Bindemittelmenge wurde jeweils so bemessen, dass keine Ausbildung von Zwillingen erfolgte, aber die gesamte Pulvermenge auf die Oberfläche der Trägerkörper aufgenommen wurde ohne dass Pulveragglomeration eintrat). Gleichzeitig wurden im selben Zeitraum 205 g des gemahlenen katalytisch aktiven Oxidmassenpulvers über eine Schüttelrinne außerhalb des Sprühkegels der Zerstäuberdüse kontinuierlich zudosiert. Während der Beschichtung wurde das zugeführte Pulver vollständig auf die Oberfläche der Trägerkörper aufgenommen. Eine Agglomeration der feinteiligen oxidischen Aktivmasse wurde nicht beobachtet.

Anschließend wurden die beschichteten Ringe 2 h bei einer Temperatur von 300°C in einem Umlufttrockenschrank gehalten (entfeuchtet).

Die dem Umlufttrockenschrank entnommenen Schalenkatalysatoren wiesen, bezogen auf ihre Gesamtmasse, einen oxidischen Aktivmassenanteil von ca. 20 Gew.-% auf. Die Aktivmassenschalendicke betrug 150 bis 250 $\mu$m.

Vergleichsbeispiel 2B

Die Herstellung des ringförmigen Schalenkatalysators V2B mit der katalytisch aktiven Oxidmasse $Mo_{12}V_3W_{1,2}Cu_{1,8}O_n$ erfolgte wie bei V1B, anstelle von 23,9 g wurden jedoch 71,8 g Kupfer(II)acetatmonohydrat (Cu-Gehalt = 31,7 Gew.-%) eingesetzt.

Vergleichsbeispiel 3B

Die Herstellung des ringförmigen Schalenkatalysators V3B mit der katalytisch aktiven Oxidmasse $Mo_{12}V_3W_{1,2}Cu_{2,4}O_n$, erfolgte wie bei V1B, anstelle von 23,9 g wurden jedoch 96,6 g Kupfer(II)acetatmonohydrat (Cu-Gehalt = 31,7 Gew.-%) eingesetzt.

Ausführungsbeispiel 1B

Die Herstellung des ringförmigen Schalenkatalysators A1B mit der katalytisch aktiven Oxidmasse $Mo_{12}V_3W_{1,2}Cu_{1,2}O_n$ erfolgte wie bei V1B, anstelle von 23,9 g wurden jedoch 47,8 g Kupfer(II)acetatmonohydrat (Cu-Gehalt = 31,7 Gew.-%) eingesetzt.

D) Testung der Schalenkatalysatoren V1B bis V3B sowie A1B als Katalysatoren für die heterogen katalysierte partielle Gasphasenoxidation von Acrolein zu Acrylsäure

Ein Reaktionsrohr (V2A Stahl; 30 mm Außendurchmesser; 2 mm Wandstärke; 26 mm Innendurchmesser; 464 cm Länge) wurde von oben nach unten wie folgt beschickt:

Abschnitt 1: 80 cm Länge
Leerrohr;

Abschnitt 2: 60 cm Länge
Vorschüttung aus Steatit-Ringen der Geometrie 7 mm x 3 mm x 4 mm (Außendurchmesser x Länge x Innendurchmesser; Steatit C 220 der Fa. CeramTec);

Abschnitt 3: 100 cm Länge

Katalysatorfestbettschüttung aus einem homogenen Gemisch bestehend aus 20 Gew.-% Steatit-Ringen der Geometrie 7 mm x 3 mm x 4 mm (Außendurchmesser x Länge x Innendurchmesser; Steatit C 220 der Fa. CeramTec) und 80 Gew.-% des jeweiligen Schalenkatalysators;

Abschnitt 4: 200 cm Länge

Katalysatorfestbettschüttung ausschließlich bestehend aus dem in Abschnitt 3 jeweils verwendeten Schalenkatalysator;

Abschnitt 5: 10 cm Länge

Nachschüttung aus denselben Steatit-Ringen wie in Abschnitt 2;

Abschnitt 6: 14 cm Länge

Katalysatorstuhl aus V2A Stahl zur Aufnahme des Katalysatorfestbetts.

Durch das jeweilige wie vorstehend beschrieben beschickte Reaktionsrohr wurde von oben nach unten durch das Reaktionsrohr strömend ein Reaktionsgasgemisch geführt, das folgende Gehalte aufwies:

4,25 Vol.-% Acrolein,
0,3 Vol.-% Propen,
0,2 Vol.-% Propan,
0,3 Vol.-% Acrylsäure,
5,15 Vol.-% $O_2$,
0,5 Vol.-% CO und $CO_2$,
7 Vol.-% $H_2O$ und
82,3 Vol.-% $N_2$.

Die Zufuhrtemperatur des Reaktionsgasgemischs (am Eingang in das Reaktionsrohr) betrug 210 °C und die Belastung des Katalysatorfestbetts (wie in der DE-A 19927624 definiert) mit Acrolein war 80 Nl/l·h.

Das Reaktionsrohr war über seine Länge (bis auf die letzten 10 cm des Leerrohres im Abschnitt 1 und die letzten 3 cm des Rohres im Abschnitt 6) jeweils von einem gerührten und von außen elektrisch beheizten Salzbad (Gemisch aus 53 Gew.-% Kaliumnitrat, 40 Gew.-% Natriumnitrit und 7 Gew.-% Natriumnitrat, 50 kg Salzschmelze) umspült (die Strömungsgeschwindigkeit am Rohr war 3 m/s). Die Salzbadtemperatur $T^B$ (°C) (mit der das Salzbad zugeführt wurde) wurde in allen Fällen so eingestellt, dass ein auf den einfachen Durchgang des Reaktionsgasgemischs durch das Katalysatorfestbett bezogener Acroleinumsatz von 99,3 mol-% resultierte. Entlang des Reaktionsrohres änderte sich die Salzbadtemperatur infolge Zuheizung nicht (es wurde vom Salzbad mehr Wärme abgestrahlt wie vom Reaktionsrohr an das Salzbad abgegeben).

Die nachfolgende Tabelle 2 zeigt die in Abhängigkeit vom eingesetzten Schalenkatalysator nach 100 Betriebsstunden resultierenden Ergebnisse:

Tabelle 2

| Schalen katalysator | $T^B$ (°C) | $U^A$ (mol-%) | $S^{AS}$ (mol-%) |
|---|---|---|---|
| V1B | 270 | 99,3 | 96,9 |
| V2B | 261 | 99,3 | 96,2 |
| V3B | 275 | 99,3 | 95,4 |
| A1B | 256 | 99,3 | 96,9 |

Die in Tabelle 2 gezeigten Ergebnisse weisen aus, dass die Stöchiometrie $Mo_{12}V_3W_{1,2}Cu_{1,2}O_n$ die höchste Aktivität aufweist. Gleichzeitig liegt diese Stöchiometrie an der Spitze bezüglich der erzielten Zielproduktselektivität.

E) Herstellung von Schalenkatalysatoren

Vergleichsbeispiel 1C

Die Herstellung des ringförmigen Schalenkatalysators V1C mit der katalytisch aktiven Oxidmasse $Mo_{12}V_3W_{1,2}Cu_{2,4}O_n$, erfolgte wie beim Vergleichsbeispiel 3B. Allerdings mit dem Unterschied, dass der während der gesamten Calcination durch das Drehrohr geleitete Gasstrom nicht 2,2 Vol.-% sondern 2,6 Vol.-% an molekularem Sauerstoff enthielt.

Ausführungsbeispiel 1C

Die Herstellung des ringförmigen Schalenkatalysators A1C mit der katalytisch aktiven Oxidmasse $Mo_{12}V_3W_{1,2}Cu_{1,2}O_n$ erfolgte wie beim Ausführungsbeispiel 1B. Allerdings mit dem Unterschied, dass der während der gesamten Calcination durch das Drohrohr geleitete Gasstrom nicht 2,2 Vol.-% sondern 2,6 Vol.-% an molekularem Sauerstoff enthielt.

F) Testung der Schalenkatalysatoren V1C sowie A1C als Katalysatoren für die heterogen katalysierte partielle Gasphasenoxidation von Acrolein zu Acrylsäure

Die Testung erfolgte wie in B). Die Gehalte des von unten durch das Reaktionsrohr strömenden Reaktionsgasgemischs waren jedoch wie folgt:

4,6 Vol.-% Acrolein,
0,1 Vol.-% Propen,
0,3 Vol.-% Acrylsäure,
5,6 Vol.-% $O_2$,
1,3 Vol.-% CO und $CO_2$,
82,1 Vol.-% $N_2$ und
6,0 Vol.-% $H_2O$.

Die Belastung des Katalysatorfestbetts (wie in der DE-A 19927624 definiert) mit Acrolein war 84 Nl/l·h.
Die nachfolgende Tabelle 3 zeigt die in Abhängigkeit vom eingesetzten Schalenkatalysator nach jeweils 100 Betriebsstunden resultierenden Ergebnisse:

Tabelle 3

| Schalen katalysator | $T^B$ (°C) | $U^A$ (mol-%) | $S^{AS}$ (mol.-%) |
|---|---|---|---|
| V1C | 260 | 99,5 | 95,8 |
| A1C | 258 | 99,5 | 96,4 |

Bemerkenswerterweise zeigt die im Thermorohr bei Verwendung des Schalenkatalysators A1C entlang des Thermorohrs in Strömungsrichtung des Reaktionsgasgemischs mittels eines Thermoelements gemessene Temperatur zwei Temperaturmaxima. Das erste Temperaturmaximum beträgt 287°C und liegt im Beschickungsabschnitt 3 bei der Position "150 cm vom unteren Reaktionsrohrende". Das zweite Temperaturmaximum beträgt 291°C und liegt im Beschickungsabschnitt 4 bei der Position "210 cm vom unteren Reaktionsrohrende".
Im Unterschied dazu zeigt die im Thermorohr bei Verwendung des Schalenkatalysators V1C entlang des Thermorohrs in Strömungsrichtung mittels eines Thermoelements gemessene Temperatur zwar ebenfalls zwei Temperaturmaxima. Das erste Temperaturmaximum (in Strömungsrichtung) beträgt in diesem Fall jedoch 290°C und das zweite Temperaturmaximum beträgt 287°C (die Positionen der Temperaturmaxima liegen an denselben Stellen wie im Fall der Verwendung des Schalenkatalysators A1C). Es überrascht, dass bei Verwendung des Schalenkatalysators A1C eine höhere Zielproduktselektivität resultiert, obwohl in diesem Fall das in Strömungsrichtung des Reaktionsgasgemischs zweite Temperaturmaximum die höhere Temperatur aufweist.
Wurde bei einer Reaktionsgasgemischzusammensetzung von

5 Vol.-% Acrolein,
0,1 Vol.-% Propen,
0,4 Vol.-% Acrylsäure,
5,5 Vol.-% $O_2$,
1,4 Vol.-% CO und $CO_2$,
80,9 Vol.-% $N_2$ und
6,7 Vol.-% $H_2O$

unter ansonsten identischen Betriebsbedingungen im Fall des Schalenkatalysators A1C die Belastung des Katalysatorfestbetts mit Acrolein auf 104 Nl/l·h erhöht, wurden nach 100 Betriebsstunden folgende Ergebnisse erhalten:

| $T^B$ (°C) | $U^A$ (mol-%) | $S^{AS}$ (mol.-%) |
|---|---|---|
| 259 | 99,5 | 96,2 |

Auch in diesem Fall liegen im Thermorohr bei den Positionen "150 cm vom unteren Reaktionsrohrende" und "210 cm vom unteren Reaktionsrohrende" Temperaturmaxima vor. Das in Strömungsrichtung erste Maximum beträgt jetzt 302°C und das in Strömungsrichtung zweite Temperaturmaximum beträgt jetzt 294°C.

Diese Ergebnisse weisen aus, dass die Stöchiometrie $Mo_{12}V_3W_{1,2}Cu_{1,2}O_n$ selbst bei einer vergleichsweise erhöhten Acroleinbelastung noch eine höhere Selektivität der Acrylsäurebildung gewährleistet, als die Stöchiometrie $Mo_{12}V_3W_{1,2}Cu_{2,4}O_n$, bei einer vergleichsweise geringeren Acroleinbelastung.

## Patentansprüche

1. Schalenkatalysator bestehend aus einem hohlzylindrischen Trägerkörper einer Länge von 2 bis 10 mm, einem Außendurchmesser von 4 bis 10 mm und einer Wanddicke von 1 bis 4 mm sowie einer auf die äußere Oberfläche des Trägerkörpers aufgebrachten Schale aus katalytisch aktiver Oxidmasse der allgemeinen Formel I,

$$Mo_{12}V_{2 \text{ bis } 4}W_{0 \text{ bis } 3}Cu_{0,8 \text{ bis } 1,5}X^1_{0 \text{ bis } 4}X^2_{0 \text{ bis } 40}O_n \qquad (I)$$

, in der die Variablen folgende Bedeutung haben:

| | |
|---|---|
| $X^1$ = | eines oder mehrere Elemente der Alkali- und Erdalkalimetalle; |
| $X^2$ = | eines oder mehrere Elemente aus der Gruppe Si, Al, Ti und Zr; und |
| n = | der stöchiometrische Koeffizient des Elementes Sauerstoff, der durch die stöchiometrischen Koeffizienten der von Sauerstoff verschiedenen Elemente sowie deren Ladungszahl in I bestimmt wird. |

2. Schalenkatalysator nach Anspruch 1, **dadurch gekennzeichnet, dass** der stöchiometrische Koeffizient des Elements W in der allgemeinen Formel I 0,2 bis 3 beträgt.

3. Schalenkatalysator nach Anspruch 1, **dadurch gekennzeichnet, dass** der stöchiometrische Koeffizient des Elements W in der allgemeinen Formel I 0,5 bis 2 beträgt.

4. Schalenkatalysator nach Anspruch 1, **dadurch gekennzeichnet, dass** der stöchiometrische Koeffizient des Elements W in der allgemeinen Formel I 0,75 bis 1,5 beträgt.

5. Schalenkatalysator nach Anspruch 1, **dadurch gekennzeichnet, dass** der stöchiometrische Koeffizient des Elements V in der allgemeinen Formel I 2,5 bis 3,5 beträgt.

6. Schalenkatalysator nach Anspruch 1, **dadurch gekennzeichnet, dass** der stöchiometrische Koeffizient des Elements Cu in der allgemeinen Formel I 1,0 bis 1,5 beträgt.

7. Schalenkatalysator nach Anspruch 1, **dadurch gekennzeichnet, dass** der hohlzylindrische Trägerkörper eine Länge von 3 bis 6 mm, einen Außendurchmesser von 4 bis 8 mm und eine Wanddicke von 1 bis 2 mm aufweist.

8. Schalenkatalysator nach Anspruch 1, **dadurch gekennzeichnet, dass** die Dicke der auf den hohlzylindrischen Trägerkörper aufgebrachten Schale aus katalytisch aktiver Oxidmasse 10 bis 1000 $\mu$m, oder 10 bis 500 $\mu$m, oder 100 bis 500 $\mu$m oder 200 bis 300 $\mu$m beträgt.

9. Verfahren zur Herstellung von Acrylsäure durch gasphasenkatalytische Oxidation von Acrolein an einem Katalysatorfestbett, **dadurch gekennzeichnet, dass** das Katalysatorfestbett einen Schalenkatalysator gemäß einem der Ansprüche 1 bis 8 umfasst.

10. Verfahren nach Anspruch 9, **dadurch gekennzeichnet, dass** die Belastung des Katalysatorfestbetts mit Acrolein 50 bis 350 Nl/l·h, oder 135 bis 250 Nl/l·h beträgt.

**11.** Verwendung von Schalenkatalysatoren gemäß einem der Ansprüche 1 bis 8 als Katalysatoren für die heterogen katalysierte partielle Gasphasenoxidation von Acrolein zu Acrylsäure.

**Claims**

**1.** An eggshell catalyst consisting of a hollow cylindrical support body of length 2 to 10 mm, external diameter 4 to 10 mm and wall thickness 1 to 4 mm, and an eggshell, applied to the outer surface of the support body, of catalytically active oxide material of the general formula I,

$$Mo_{12}V_{2 \text{ to } 4}W_{0 \text{ to } 3}Cu_{0.8 \text{ to } 1.5}X^1_{0 \text{ to } 4} X^2_{0 \text{ to } 40}O_n \qquad (I)$$

in which the variables are each defined as follows:

$X^1$ = one or more elements of the alkali metals and alkaline earth metals;
$X^2$ = one or more elements from the group of Si, Al, Ti and Zr; and
n = the stoichiometric coefficient of the element oxygen, which is determined by the stoichiometric coefficients of the elements other than oxygen and the charges thereof in I.

**2.** The eggshell catalyst according to claim 1, wherein the stoichiometric coefficient of the element W in the general formula I is 0.2 to 3.

**3.** The eggshell catalyst according to claim 1, wherein the stoichiometric coefficient of the element W in the general formula I is 0.5 to 2.

**4.** The eggshell catalyst according to claim 1, wherein the stoichiometric coefficient of the element W in the general formula I is 0.75 to 1.5.

**5.** The eggshell catalyst according to claim 1, wherein the stoichiometric coefficient of the element V in the general formula I is 2.5 to 3.5.

**6.** The eggshell catalyst according to claim 1, wherein the stoichiometric coefficient of the element Cu in the general formula I is 1.0 to 1.5.

**7.** The eggshell catalyst according to claim 1, **characterized in that** the hollow cylindrical support body has a length of 3 to 6 mm, an external diameter of 4 to 8 mm and a wall thickness of 1 to 2 mm.

**8.** The eggshell catalyst according to claim 1, wherein the thickness of the eggshell of catalytically active oxide material applied to the hollow cylindrical support body is 10 to 1000 $\mu$m, or 10 to 500 $\mu$m, or 100 to 500 $\mu$m, or 200 to 300 $\mu$m.

**9.** A process for preparing acrylic acid by catalytic oxidation of acrolein in the gas phase over a fixed catalyst bed, wherein the fixed catalyst bed comprises an eggshell catalyst according to any of claims 1 to 8.

**10.** The process according to claim 9, wherein the hourly space velocity of acrolein on the fixed catalyst bed is 50 to 350 l (STP) /l·h, or 135 to 250 l (STP) /l·h.

**11.** The use of eggshell catalysts according to any of claims 1 to 8 as catalysts for the heterogeneously catalyzed partial gas phase oxidation of acrolein to acrylic acid.

**Revendications**

**1.** Catalyseur à enveloppe constitué par un corps support cylindrique creux d'une longueur de 2 à 10 mm, d'un diamètre extérieur de 4 à 10 mm et d'une épaisseur de paroi de 1 à 4 mm, ainsi que par une enveloppe d'une masse d'oxyde catalytiquement active, appliquée sur la surface extérieure du corps support de formule générale I

$$Mo_2V_{2 \text{ à } 4}W_{0 \text{ à } 3}Cu_{0,8 \text{ à } 1,5}X^1_{0 \text{ à } 4}X^2_{0 \text{ à } 40}O_n \qquad (I)$$

dans laquelle les variables ont la signification suivante :

X$^1$ = un ou plusieurs éléments des métaux alcalins et alcalino-terreux ;
X$^2$ = un ou plusieurs éléments du groupe constitué par Si, Al, Ti et Zr ; et
n = le coefficient stoechiométrique de l'élément oxygène, qui est déterminé par les coefficients stoechiométriques des éléments différents de l'oxygène et leur nombre de charge dans I.

2. Catalyseur à enveloppe selon la revendication 1, **caractérisé en ce que** le coefficient stoechiométrique de l'élément W dans la formule générale I est de 0,2 à 3.

3. Catalyseur à enveloppe selon la revendication 1, **caractérisé en ce que** le coefficient stoechiométrique de l'élément W dans la formule générale I est de 0,5 à 2.

4. Catalyseur à enveloppe selon la revendication 1, **caractérisé en ce que** le coefficient stoechiométrique de l'élément W dans la formule générale I est de 0,75 à 1,5.

5. Catalyseur à enveloppe selon la revendication 1, **caractérisé en ce que** le coefficient stoechiométrique de l'élément V dans la formule générale I est de 2,5 à 3,5.

6. Catalyseur à enveloppe selon la revendication 1, **caractérisé en ce que** le coefficient stoechiométrique de l'élément Cu dans la formule générale I est de 1,0 à 1,5.

7. Catalyseur à enveloppe selon la revendication 1, **caractérisé en ce que** le corps support cylindrique creux présente une longueur de 3 à 6 mm, un diamètre extérieur de 4 à 8 mm et une épaisseur de paroi de 1 à 2 mm.

8. Catalyseur à enveloppe selon la revendication 1, **caractérisé en ce que** l'épaisseur de l'enveloppe de masse d'oxyde catalytiquement active appliquée sur le corps cylindrique creux est de 10 à 1 000 $\mu$m, ou de 10 à 500 $\mu$m, ou de 100 à 500 $\mu$m, ou de 200 à 300 $\mu$m.

9. Procédé de fabrication d'acide acrylique par oxydation catalytique en phase gazeuse d'acroléine sur un lit catalytique fixe, **caractérisé en ce que** le lit catalytique fixe comprend un catalyseur à enveloppe selon l'une quelconque des revendications 1 à 8.

10. Procédé selon la revendication 9, **caractérisé en ce que** le chargement du lit catalytique fixe avec l'acroléine est de 50 à 350 Nl/l·h, ou de 135 à 250 Nl/l·h.

11. Utilisation de catalyseurs à enveloppe selon l'une quelconque des revendications 1 à 8 en tant que catalyseurs pour l'oxydation catalytique hétérogène partielle en phase gazeuse d'acroléine en acide acrylique.

## Fig.1

# Fig.2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 714700 A
- DE 19927624 A
- EP 0015569 A
- DE 10360057 A

- WO 9511081 A
- WO 2006094766 A
- DE 102007028333 A
- EP 714700 B